# EUROPÄISCHE PATENTSCHRIFT

(11) **EP 3 880 644 B1**
(45) Veröffentlichungstag und Bekanntmachung des Hinweises auf die Patenterteilung: **06.09.2023**
(21) Anmeldenummer: 19702042.3
(22) Anmeldetag: 23.01.2019
(51) Int. Cl.: C07C 67/08, C07C 69/67

(54) **VERFAHREN ZUR HERSTELLUNG VON VERKAPPTEN 3-HYDROXYCARBONSÄUREN SOWIE DEREN SALZEN UND ESTERN**
PROCESS FOR PREPARING CAPPED 3-HYDROXYCARBOXYLIC ACIDS AND THEIR SALTS AND ESTERS
PROCÉDÉ DE PRÉPARATION D'ACIDES 3-HYDROXYCARBOXYLIQUES COIFFÉS ET DE LEURS SELS ET ESTERS

(30) Priorität: 17.01.2019 WO PCT/EP2019/051123
(43) Veröffentlichungstag der Anmeldung: 22.09.2021
(73) Patentinhaber: KetoLipix Therapeutics GmbH, 20459 Hamburg (DE)
(72) Erfinder: LOCHMANN, Dirk, 58453 Witten (DE); REYER, Sebastian, 58453 Witten (DE); STEHR, Michael, 58453 Witten (DE)
(74) Vertreter: Strehlke, Ingo Kurt
(86) Internationale Anmeldenummer: PCT/EP2019/051543
(87) Internationale Veröffentlichungsnummer: WO 2020/147981

(56) Entgegenhaltungen:
- WO-A1-2014/099300
- WO-A1-2017/213999
- DE-T2- 3 873 016
- US-A- 2 026 985
- US-A1- 2005 014 974
- ZHANG ET AL.: "Cofactor Recycling Mechanism in Asymmetric Biocatalytic reduction of Carbonyl Compounds Mediated by Yeast: Which is the efficient Electron Donor?", CHEMISTRY - A EUROPEAN JOURNAL, Bd. 9, 8. April 2003 (2003-04-08), Seiten 3604-3610, XP002790556,
- EVANS ET AL.: "Regio- and enantiospecific Rhodium-Catalyzed Allylic Etherification Reactions Using Copper(I) Alkoxides: influence of the Copper Halide Salt on Selectivity", J. AM. CHEM. SOC., Bd. 124, 10. Juli 2002 (2002-07-10), Seiten 7882-7883, XP002790557,
- Xiao-Chuan Liu ET AL: "COMMUNICATION TO THE EDITOR Chemoenzymatic Construction of a Four-Component Ugi Combinatorial Library", , 20. August 2000 (2000-08-20), XP055644392, Gefunden im Internet: URL:https://onlinelibrary.wiley.com/doi/10 .1002/1097-0290%2820000820%2969%3A4%3C457% 3A%3AAID-BIT12%3E3.0.CO%3B2-L [gefunden am 2019-11-20]
- BALLAUS O: "Studien zum Raman-Effekt", MONATSHEFTE FUER CHEMIE UND VERWANDTE TEILE ANDEREN WISSENSCHAFTEN, SPRINGER, VIENNA, AT, Bd. 74, 1. Januar 1943 (1943-01-01), Seiten 85-91, XP009517358, ISSN: 0343-7329
- AUTENRIETH W: "UEBER EINFACHE UND GEMISCHTE SAEUREANHYDRIDE", CHEMISCHE BERICHTE, VCH, DE, Bd. 34, 14. Januar 1901 (1901-01-14), Seiten 177-187, XP009034916, ISSN: 0009-2940

## Beschreibung

Die vorliegende Erfindung betrifft das Gebiet der Ketokörper und des damit zusammenhängenden Stoffwechsels sowie die Therapie von damit im Zusammenhang stehenden Erkrankungen.

Insbesondere betrifft die vorliegende Erfindung ein Verfahren zur Herstellung von verkappten oder blockierten 3-Hydroxybuttersäuren sowie deren Salzen und Estern.

Im menschlichen Energiestoffwechsel ist Glucose der kurzfristig zur Verfügung stehende Energieträger, welcher in den Mitochondrien unter Freisetzung von Wasser und Kohlendioxid zu Energie verstoffwechselt wird. Bereits durch die Schlafperiode während der Nacht sind aber die Glycogenspeicher der Leber geleert. Jedoch benötigen vor allem das menschliche zentrale Nervensystem (ZNS) und das Herz eine permanente Energieversorgung.

Die physiologische Alternative zu Glucose, welche vor allem dem zentralen Nervensystem zur Verfügung steht, sind die sogenannten Ketokörper (synonym auch als Ketonkörper bezeichnet oder Englisch auch als "*Keton Bodies*" bezeichnet).

Der Begriff der Ketokörper ist insbesondere eine Sammelbezeichnung für drei Verbindungen, welche vor allem in katabolen Stoffwechsellagen (wie z. B. bei Hunger, Reduktionsdiät oder kohlenhydratarmer Ernährung) gebildet werden und unter Umständen zu einer Ketose führen. Unter den Begriff der Ketokörper fasst man insbesondere die drei Verbindungen Acetoacetat (synonym auch Acetacetat genannt) und Aceton sowie 3-Hydroxybuttersäure (nachfolgend synonym auch als beta-Hydroxybuttersäure oder BHB oder 3-BHB bezeichnet) bzw. deren Salz (d. h. 3-Hydroxybutyrat oder beta-Hydroxybutyrat) zusammen, wobei letztere Verbindung die bedeutendste der drei vorgenannten Verbindungen ist. 3-Hydroxybuttersäure bzw. deren Salz kommt physiologisch als (R)-Enantiomer vor, d. h. als (R)-3-Hydroxybuttersäure (synonym auch (3R)-3-Hydroxybuttersäure genannt, um das Chiralitätszentrum in 3-Position hervorzuheben) bzw. deren Salz.

Diese Ketokörper werden auch in großer Zahl beim Fasten oder Hungern physiologisch aus im Körper eingelagerten Lipiden durch Lipolyse bereitgestellt und ersetzen den Energieträger Glucose fast vollständig.

Die Ketokörper werden in der Leber aus Acetyl-Coenzym A (= Acetyl-CoA) gebildet, welches aus der beta-Oxidation stammt; sie stellen eine transportable Form des Acetyl-Coenzyms A im menschliche Körper dar. Zur Verwertung der Ketokörper müssen sich Gehirn und Muskeln aber zunächst umstellen, indem sie Enzyme exprimieren, welche zur Rückwandlung von Ketokörpern in Acetyl-Coenzym A benötigt werden. Insbesondere in Hungerzeiten tragen die Ketokörper einen beträchtlichen Anteil zur Energiegewinnung bei. So ist es beispielsweise dem Gehirn nach einiger Zeit möglich, mit nur einem Drittel der Tagesmenge an Glucose auszukommen.

Physiologisch erfolgt die Synthese der Ketokörper aus zwei Molekülen aktivierter Essigsäure in Form von Acetyl-Coenzym A, dem normalen Zwischenprodukt des Fettsäureabbaus, wobei zunächst mit Hilfe der Acetyl-Coenzym A-Acetyltransferase das Acetoacetyl-Coenzym A gebildet wird, welches unter Verwendung einer weiteren Acetyl-Coenzym A-Einheit und des Enzyms HMG-CoA-Synthase zum Zwischenprodukt 3-Hydroxy-3-methyl-glutaryl-CoA (HMG-CoA) verlängert wird, wobei schließlich die HMG-CoA-Lyase das Acetoacetat abspaltet. Diese drei Schritte finden ausschließlich in den Mitochondrien der Leber statt (Lynenzyklus), wobei 3-Hydroxybutyrat schließlich im Zytosol durch die D-beta-Hydroxybutyrat-Dehydrogenase entsteht. HMG-CoA ist außerdem ein Endprodukt beim Abbau der Aminosäure Leucin, während Acetoacetat beim Abbau der Aminosäuren Phenylalanin und Tyrosin entsteht.

Durch spontane Decarboxylierung entsteht aus Acetoacetat Aceton; es ist gelegentlich im Atem von Diabetikern und Diäthaltenden wahrzunehmen. Es kann vom Körper nicht weiterverwendet werden. Der Anteil von Aceton an den Ketokörpern ist allerdings gering.

Acetoacetat wird also reduktiv in die physiologisch relevante Form der 3-Hydroxybuttersäure bzw. des 3-Hydroxybutyrats überführt, kann aber auch unter Kohlenstoffdioxidfreisetzung in das physiologisch unbrauchbare Aceton zerfallen, was bei einer schweren Ketose, einer Ketoacidose (z. B. bei Diabetes mellitus Typ 1-Patienten ohne Insulinsubstitution), im Urin und in der Ausatemluft nachweisbar und olfaktorisch wahrnehmbar ist.

3-Hydroxybuttersäure wird derzeit im Bereich des Kraftsports als Natrium-, Magnesium- oder Calcium-Salz eingesetzt und in den Handel gebracht.

Jedoch ist 3-Hydroxybuttersäure für den Menschen evolutionär nicht oder in nur sehr geringer Menge bekannt, da Pflanzen keine 3-Hydroxybuttersäure produzieren und 3-Hydroxybuttersäure im tierischen Organismus nur bei toten ausgezehrten Tieren in der Ketose vorkommt, so dass 3-Hydroxybuttersäure bei peroraler Verabreichung Brechreiz auslöst. 3-Hydroxybuttersäure in Form der freien Säure sowie deren Salzen schmeckt zudem stark bitter und kann schweres Erbrechen und Übelkeit hervorrufen.

Zudem können Patienten, vor allem Neugeborene, aber auch Erwachsene größere Mengen an Salzen der 3-Hydroxybuttersäure nicht permanent verkraften, da diese Verbindungen nierenschädigend wirken können.

Außerdem ist die Plasmahalbwertszeit von 3-Hydroxybuttersäure und deren Salzen derart gering, dass selbst bei Einnahme von mehreren Gramm die Ketose nur für ca. drei bis vier Stunden vorhält, d. h. Patienten können insbesondere während der Nacht daher nicht von einer Therapie mit 3-Hydroxybuttersäure oder deren Salzen kontinuierlich profitieren. Bei Stoffwechselerkrankungen kann dies zu lebensbedrohlichen Situationen führen.

Daher werden im Fall der Therapie derartiger Stoffwechselerkrankungen heute sogenannte mittelkettige Triglyceride, sogenannte MCTs, für die ketogene Therapie eingesetzt, d. h. es wird die metabolische Umwandlung von Capron-, Capryl- und Caprinsäure (d. h. von gesättigten linearen C₆-, Cs- und C₁₀-Fettsäuren) aus den korrespondierenden Triglyceriden beabsichtigt.

Grundsätzlich stellt aber aus pharmazeutischer und klinischer Hinsicht 3-Hydroxybuttersäure demgegenüber ein wirksameres pharmazeutisch-pharmakologisches Zielmolekül, welches nach den Erkenntnissen des Standes der Technik prinzipiell für die Therapie einer Vielzahl von Erkrankungen zum Einsatz kommen könnte, aber aufgrund seiner mangelnden physiologischen Kompatibilität dort nicht zum Einsatz kommen kann (z. B. bei Erkrankungen im Zusammenhang mit einer Störung des Energiestoffwechsels, insbesondere Ketokörperstoffwechsels, oder neurodegenerativen Erkrankungen wie Demenz, Morbus Alzheimer, Morbus Parkinson etc., Fettstoffwechselerkrankungen usw.).

Die nachfolgende Tabelle veranschaulicht rein beispielhaft, aber keinesfalls beschränkend potentielle Therapiemöglichkeiten bzw. mögliche Indikationen für den Wirkstoff 3-Hydroxybuttersäure.

| **Indikation** | **Therapeutischer Effekt** |
|---|---|
| Schädel-Hirn-Trauma | Unter BHB sinkt die Apoptose und Nekrose-Rate von Nervenzellen. |
| Schlaganfall | Unter BHB sinkt die Apoptose und Nekrose-Rate von Nervenzellen. |
| Refeeding-Syndrom | Bei Anorexie, Absetzen enteraler oder parenteraler Ernährung und nach langen Hungerperioden kann der Konsum von Stärke oder Glucose zum Tod führen (siehe auch WHO-Schema Erdnusspaste). BHB kann hier als Therapeutikum zum schnelleren Erlangen einer normalen Nahrungsaufnahme eingesetzt werden. |
| Appetitzügler | BHB unterdrückt im Zentralnervensystem (ZNS) das Hungergefühl. |
| Epilepsie | Herkömmliche ketogene Diät zur signifikanten Reduzierung der Häufigkeit von Krampfanfällen hat extrem schlechte Patienten-Verträglichkeit. BHB bietet hier eine unmittelbar wirksame Alternative. |
| Morbus Alzheimer, Demenz | Unter BHB zeigen Patienten eine bessere kognitive Leistung. BHB ist auch für die Prävention von neurodegenerativen Erkrankungen wirksam. |
| Störungen der Fettsäureoxidation (z. B. Electron-Transfer-Protein Defekt) | Ausgleich eines Nährstoffmangels bei Defekt im Energiestoffwechsel. |

Daher ist es aus pharmazeutischer und klinischer Hinsicht wünschenswert, wirksame Präkursoren oder Metabolite auffinden zu können, welche physiologisch einen direkten oder indirekten Zugang zu 3-Hydroxybuttersäure oder deren Salzen ermöglichen, insbesondere im physiologischen Stoffwechsel des menschlichen oder tierischen Körpers.

Folglich hat es im Stand der Technik nicht an Versuchen gefehlt, physiologisch geeignete Präkursoren oder Metaboliten für 3-Hydroxybuttersäure bzw. deren Salze aufzufinden. Bislang wurden im Stand der Technik jedoch keine effizienten diesbezüglichen Verbindungen aufgefunden. Auch ist ein diesbezüglicher Zugang zu solchen Verbindungen nach dem Stand der Technik bislang nicht bzw. nicht ohne Weiteres möglich.

Die wissenschaftliche Publikation gemäß Zhang et al.: "Cofactor Recycling Mechanism in Asymmetric Biocatalytic Reduction of Carbonyl Compounds Mediated by Yeast: Whic is the efficient Electron Donor?", Chemistry - A European Journal, Bd. 9, 8. April 2003, Seiten 3604-3610, XP002790556 betrifft den Mechanismus der Cofaktorregeneration bei der durch Hefe katalysierten asymmetrischen Reduktion unter Verwendung einer quantitativen Isotopenverfolgung nahe der natürlichen Häufigkeit.

Darüber hinaus betrifft die wissenschaftliche Publikation gemäß Evans et al.: "Regio- and enantiospecific Rhodium-Catalyzed Allylic Etherification Reactions Using Copper(I) Alkoxides: influence of the Copper Halide Salt on Selectivity", J. Am. Chem. Soc., Bd. 124, 10. Juli 2002, Seiten 7882-7883, XP002790557 regio- und enantiospezifische Rhodium-katalysierte Allylveretherung von acyclischen unsymmetrischen Allylalkoholderivaten unter Verwendung von Kupfer (I)-Alkoxiden abgeleitet von primären, sekundären und tertiären Alkoholen, wobei die Auswahl des Kupfer (I)-Salzes entscheidend für den Erhalt einer hohen Stereospezifität ist.

Weiterhin betrifft die DE 38 73 016 T2 einen δ-Valerolactonring enthaltende, optisch aktive Verbindung, welche als ferroelektrischer Flüssigkristall oder als Additiv zu einem ferroelektrischen Flüssigkristall verwendet werden kann sowie eine Flüssigkristallzusammensetzung, welche diese optische aktive Verbindung enthält.

Ferner betrifft die WO 2017/2013999 A1 Fettsäure-β-Hydroxyester-Verbindungen, Fettsäureester von Butandiol und pharmazeutisch verträgliche Salze davon sowie pharmazeutische Zusammensetzungen mit einer oder mehreren Fettsäure-β-Hydroxyester-Verbindungen und/oder einem oder mehreren Fettsäureestern von Butandiol. Weiterhin betrifft dieses Dokument ein Verfahren zur Behandlung eines Patienten durch Verabreichung eines oder mehrerer Ester an den Patienten sowie Kits, welche ein oder mehrere der betreffenden Ester enthalten.

Des Weiteren betrifft die wissenschaftliche Publikation gemäß Xiao-Chuan Liu et al.: "Communication to the Editor Chemoenzymafic Construction of a Four-Component Ugi Combinatorial Library", 20. August 2000, XP055644392 die chemoenzymatische Herstellung einer neunteiligen Ugi-Kondensationsbibliothek, wobei die Carbonsäure- und Aminvorläufer auf 3-Hydroxybutyrat bzw. 4-Amino-1-butanol basieren und selektiv unter Verwendung einer Vielzahl von durch Schweinelipase katalysierten Acyldonoren acyliert werden.

Weiterhin betrifft die WO 2014/099300 A1 druckempfindliche Haftklebstoffe (*pressure-sensitive adhesives*)*,* welche unter typischen Verwendungsbedingungen gute Leistungseigenschaften aufweisen sollen und welche unter basischen Bedingungen leicht abgebaut und/oder entfernt werden können, wobei die Haftklebstoffe ein copolymerisches (meth)acrylbasiertes elastomeres Material enthält, welches aus einem polymerisierbaren Material hergestellt ist und ein (Meth)acrylatmonomer enthält, welches sowohl eine (Meth)acryloylgruppe als auch eine Esterbindung aufweist, welche nicht Teil der (Meth)acryloylgruppe ist.

Überdies betrifft die US 2 026 985 A die Herstellung von Anhydriden von Fettsäuren und substituierten Fettsäuren.

Darüber hinaus betrifft die wissenschaftliche Publikation gemäß Ballaus O: "Studien zum Raman-Effekt", Monatshefte für Chemie und Verwandte Teile anderen Wissenschaften, Springer, Vienna, AT, Bd. 74, 1. Januar 1943, Seiten 85-91, XP009517358 Raman-Spektren von 20 verschiedenen Monocarbonsäure-Estern und eine entsprechende Analyse und Diskussion der für die C=O-Bindung beobachteten Frequenzwerte.

Schließlich betrifft die wissenschaftliche Publikation gemäß Autenrieth W.: "Über einfache und gemischte Säureanhydride", Chemische Berichte, VCH, DE, Bd. 34, 14. Januar 1901, Seiten 177-187, XP009034916 ein Verfahren zur Herstellung von gemischten Essigsäureanhydriden.

Das der vorliegenden Erfindung zugrundeliegende Problem liegt also in der Bereitstellung eines effizienten Herstellungsverfahrens von physiologisch geeigneten bzw. physiologisch kompatiblen Präkursoren und/oder Metaboliten von 3-Hydroxybuttersäure (d. h. beta-Hydroxybuttersäure bzw. BHB bzw. 3-BHB) oder deren Salzen.

Ein solches Verfahren soll insbesondere die betreffenden BHB-Präkursoren und/oder BHB-Metaboliten in effizienter Weise zugänglich machen, insbesondere auch in größeren Mengen und ohne nennenswerte Mengen an toxischen Nebenprodukten.

In vollkommen überraschender Weise hat die Anmelderin nunmehr herausgefunden, dass verkappte oder blockierte 3-Hydroxybuttersäuren sowie deren Salze und Ester, insbesondere die Ester verkappter oder blockierter 3-Hydroxybuttersäuren, einen effizienten und physiologisch wirksamen bzw. physiologisch kompatiblen Präkursor und/oder Metaboliten für den Ketokörper 3-Hydroxybuttersäure bzw. deren Salze darstellen, und hat in diesem Zusammenhang ein effizientes Herstellungsverfahren für diese Verbindungen auffinden bzw. entwickeln können, welches einen direkten und wirksamen, insbesondere ökonomischen wie auch großtechnisch umsetzbaren Zugang zu diesen Verbindungen ermöglicht.

Zur Lösung des zuvor geschilderten Problems schlägt die vorliegende Erfindung daher ein Verfahren zur Herstellung von verkappter (blockierter) 3-Hydroxybuttersäure (beta-Hydroxybuttersäure, BHB bzw. 3-BHB) oder deren Salzen oder Estern gemäß Anspruch 1 vor; weitere, insbesondere besondere und/oder vorteilhafte Ausgestaltungen des erfindungsgemäßen Verfahrens sind Gegenstand der diesbezüglichen Verfahrensunteransprüche.

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere relativen Mengen- oder Gewichtsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder aber einzelfallbedingt - von den nachfolgend angeführten Bereichsangaben erforderlichenfalls abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung ist ein Verfahren zur Herstellung von verkappter 3-Hydroxybuttersäure oder deren Salzen oder Estern,
wobei mindestens eine Verbindung der allgemeinen Formel (I)

   CH₃-CH(OH)-CH₂-C(O)OR¹ (I)
wobei in der allgemeinen Formel (I) der Rest R¹ Wasserstoff oder Methyl oder Ethyl darstellt,
mit mindestens einem Carbonsäureanhydrid der allgemeinen Formel (II)

   R²-C(O)-O-C(O)-R³ (II)
wobei in der in der allgemeinen Formel (II) die Reste R² und R³, jeweils unabhängig voneinander, ein lineares oder verzweigtes, gesättigtes oder ein- oder mehrfach ungesättigtes C₃-C₂₁-Alkyl darstellen,
umgesetzt wird, gegebenenfalls gefolgt von einer Hydrolyse für den Fall, dass R¹ Wasserstoff darstellt,
wobei die Umsetzung der mindestens einen Verbindung der allgemeinen Formel (I) mit dem mindestens einen Carbonsäureanhydrid der allgemeinen Formel (II) in Abwesenheit von Lösemitteln und/oder ohne jedwedes Lösemittel durchgeführt wird,
so dass als Reaktionsprodukt ein oder mehrere verkappte 3-Hydroxybuttersäuren und/oder deren Salze und/oder Ester der allgemeinen Formel (III)

   CH₃-CH[O-C(O)R⁵]-CH₂-C(O)OR⁴ (III)
wobei in der allgemeinen Formel (III) der Rest R⁴ Methyl oder Ethyl oder ein Metallion, insbesondere Alkali- oder Erdalkalimetallion, darstellt und der Rest R⁵ einen Rest R² oder R³ jeweils mit der zuvor angegebenen Bedeutung darstellt,
erhalten wird/werden.

Wie zuvor ausgeführt, hat die Anmelderin nämlich vollkommen überraschend herausgefunden, dass die auf diese Weise hergestellten verkappten (= blockierten) 3-Hydroxybuttersäuren oder deren Salze oder Ester effiziente, da physiologisch verträgliche Präkursoren und/oder Metabolite der freien 3-Hydroxybuttersäure bzw. deren Salzen darstellen, welche pharmazeutisch bzw. klinisch auch in größeren Mengen zum Einsatz kommen können, da sie physiologisch kompatibel sind.

Die vorgenannten verkappten (= blockierten) 3-Hydroxybuttersäuren oder deren Salze oder Ester, welche durch das erfindungsgemäße Herstellungsverfahren erstmals in effizienter Weise zugänglich sind, stellen eine physiologisch und pharmakologisch relevante Alternative zu der freien 3-Hydroxybuttersäure bzw. deren Salzen dar.

Die Herstellung derartiger Verbindungen mittels herkömmlicher organischer Synthese ist komplex und aufwendig, da 3-Hydroxybuttersäure verstärkt zur Polymerisation und anderen unerwünschten Nebenreaktionen (z. B. Wasserabspaltung, Zersetzung etc.) neigt. Im Rahmen der vorliegenden Erfindung konnte erstmals ein effizient arbeitendes Herstellungsverfahren bereitgestellt werden, mit welchem sich verkappte (blockierte) 3-Hydroxybuttersäuren sowie deren Salze und Ester ohne unerwünschte Nebenreaktionen herstellen lassen, insbesondere einstufig.

Das erfindungsgemäße Verfahren ermöglicht somit erstmalig die Bereitstellung untoxischer verkappter (blockierter) 3-Hydroxybuttersäuren sowie deren Salzen und Estern aus an sich bekannten, kommerziell verfügbaren und vor allem physiologisch unbedenklichen Komponenten bzw. Edukten (Ausgangsverbindungen). Die resultierenden verkappten 3-Hydroxybuttersäuren sowie deren Salze und Ester können physiologisch, insbesondere im Magen und/oder im Darm, aufgespalten werden und das Zielmolekül "3-Hydroxybuttersäure" bzw. deren Salze als Wirkstoff bzw. Wirkkomponente freisetzen bzw. generieren.

Darüber hinaus weisen die vorgenannten verkappten (blockierten) 3-Hydroxybuttersäuren sowie deren Salze und Ester auch einen akzeptablen Geschmack auf, um eine Kompatibilität auch bei oraler Verabreichung größerer Mengen über einen längeren Zeitraum zu gewährleisten (z. B. Verabreichung von 50 g Tagesdosis oder mehr).

Schließlich zeigen Untersuchungen der Anmelderin, dass die erfindungsgemäßen verkappte (blockierte) 3-Hydroxybuttersäure bzw. deren Salze oder Ester nicht nur selbst effiziente Präkursoren bzw. Metabolite der freien Hydroxybuttersäure bzw. deren Salze darstellen, sondern auch als Edukte für die Synthese weiterer Präkursoren bzw. Metabolite der freien Hydroxybuttersäure bzw. deren Salze (z. B. Glyceride) verwendet werden können.

Gleichermaßen ermöglicht es das erfindungsgemäße Herstellungsverfahren, die verkappten (blockierten) 3-Hydroxybuttersäuren sowie deren Salze und Ester frei von toxischen Verunreinigungen bereitzustellen.

Darüber hinaus kann bei entsprechenden Ausgangsmaterialien die Herstellung auch enantioselektiv durchgeführt werden. So ermöglicht es beispielsweise das erfindungsgemäße Herstellungsverfahren, die biologisch relevante Form, d. h. das (R)-Enantiomer anzureichern, insbesondere durch Enzymkatalyse, um bei oraler Verabreichung das renale System von Patienten nicht zu belasten (d. h. Elimination über die Nieren). Grundsätzlich ist es aber auch möglich und kann es unter bestimmten Voraussetzungen zweckdienlich sein, das (S)-Enantiomer anzureichern.

Darüber hinaus ist das erfindungsgemäße Herstellungsverfahren, einschließlich optionaler Weiterverarbeitungs- bzw. Aufreinigungsverfahrensschritte, wirtschaftlich bzw. ökonomisch betreibbar und auch großtechnisch umsetzbar.

Insbesondere verwendet das erfindungsgemäße Herstellungsverfahren kommerziell verfügbare Edukte und ermöglicht darüber hinaus eine relativ einfache Verfahrensführung auch bei großtechnischer Umsetzung.

Im Gegensatz zu herkömmlichen Herstellungsverfahren des Standes der Technik kommt das erfindungsgemäße Herstellungsverfahren ohne komplexe Edukte aus und verläuft nur einstufig. Dennoch werden im Rahmen des erfindungsgemäßen Herstellungsverfahrens exzellente Ausbeuten erzielt, wobei in gleicher Weise die Bildung von Nebenprodukten minimiert bzw. vermieden wird.

Darüber hinaus ist das erfindungsgemäßen Verfahren einfach und wirtschaftlich. Insbesondere wird das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*); folglich sind die erhaltenen Reaktionsprodukte nicht mit Lösemittel verunreinigt und es muss kein Lösemittel nach Durchführung des Verfahrens bzw. der Reaktion aufwendig und energieträchtig entfernt und entsorgt bzw. rezykliert werden. Es werden zudem auch keine toxischen Nebenprodukte gebildet.

Das nachfolgende allgemeine Reaktionsschema veranschaulicht das erfindungsgemäße Herstellungsverfahren (wobei in diesem die Reste R¹, R², R³, R⁴ und R⁵ die zuvor angegebene Bedeutung haben und für den Fall der freien Säure, d. h. R¹ = H, als Edukt der zwischengeschaltete Hydrolyseschritt nicht dargestellt ist):

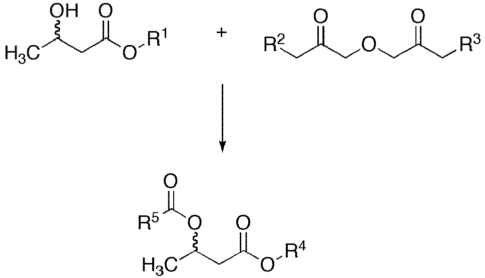

Gemäß einer besonderen Ausführungsform der vorliegenden Erfindung kann die Verbindung der allgemeinen Formel (I) entweder in racemischer Form oder aber in Form des (R)-Enantiomers eingesetzt werden. Die (R)-Konfiguration bezieht sich auf das chirale Kohlenstoffatom in 3-Position der Verbindung der allgemeinen Formel (I).

Gemäß einer besonderen Ausführungsform ist die Verbindung der allgemeinen Formel (I) ein Ester (d. h. in der obigen allgemeinen Formel (I) stellt der Rest R¹ keinen Wasserstoff dar bzw. stellt der Rest R¹ Methyl oder Ethyl dar).

Gemäß einer bevorzugten Ausführungsform des erfindungsgemäßen Herstellungsverfahrens kann in der obigen allgemeinen Formel (I) der Rest R¹ Ethyl darstellen. Mit anderen Worten wird bei dieser bevorzugten Ausführungsform als Verbindung der allgemeinen Formel (I) 3-Hydroxybuttersäureethylester (Ethyl-3-hydroxybutyrat) der Formel CH₃-CH(OH)-CH₂-C(O)OC₂H₅ eingesetzt.

Für diese bevorzugte Ausführungsform, wonach die Verbindung der allgemeinen Formel (I) ein Ethylester ist, kann das folgenden Reaktionsschema veranschaulichend für den Reaktionsverlauf bzw. -ablauf herangezogen werden (wobei in diesem Reaktionsschema die Reste R², R³und R⁵ die zuvor angegebene Bedeutung haben):

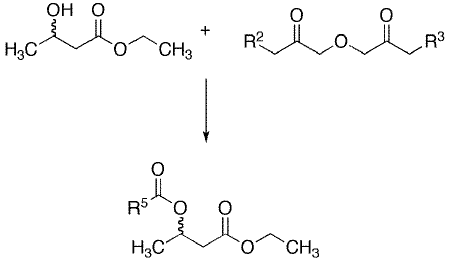

Sofern gemäß einer alternativen besonderen Ausführungsform die Verbindung der allgemeinen Formel (I) ein Säure ist (d. h. in der obigen allgemeinen Formel (I) stellt der Rest R¹ Wasserstoff dar), kann das folgenden Reaktionsschema veranschaulichend für den Reaktionsverlauf bzw. -ablauf herangezogen werden (wobei in diesem Reaktionsschema die Reste R², R³und R⁵ die zuvor angegebene Bedeutung haben), wobei die Darstellung rein beispielhaft eine saure oder neutrale Hydrolyse zeigt, welche zur freien Säure führt (wobei grundsätzlich auch eine basische Hydrolyse möglich ist, welche zu den Salzen der Säure führt):

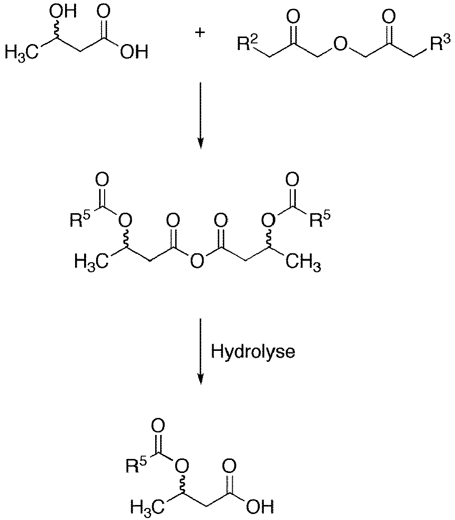

Wie zuvor bereits ausgeführt, kann für den Fall, dass die Verbindung der allgemeinen Formel (I) ein Säure ist (d. h. in der obigen allgemeinen Formel (I) der Rest R¹ Wasserstoff darstellt), die Hydrolyse grundsätzlich entweder sauer oder basisch, insbesondere basisch und/oder in Gegenwart von Metallionen, insbesondere Alkali- oder Erdalkalimetallionen, durchgeführt werden.

Gemäß einer besonderen Ausführungsform kann als Carbonsäureanhydrid der allgemeinen Formel (II) eine Verbindung eingesetzt wird, bei welcher die Reste R² und R³ identisch sind. Mit anderen Worten kann gemäß dieser besonderen Ausführungsform als Carbonsäureanhydrid der allgemeinen Formel (II) ein symmetrisches Carbonsäureanhydrid eingesetzt werden.

Alternativ hierzu kann gemäß einer anderen besonderen Ausführungsform als Carbonsäureanhydrid der allgemeinen Formel (II) eine Verbindung eingesetzt werden, bei welcher die Reste R² und R³ voneinander verschieden sind. Mit anderen Worten kann gemäß dieser alternativen besonderen Ausführungsform als Carbonsäureanhydrid der allgemeinen Formel (II) ein unsymmetrisches Carbonsäureanhydrid eingesetzt werden.

Was die Umsetzungstemperaturen anbelangt, so können diese in weiten Bereichen variieren: Insbesondere kann die Umsetzung der mindestens einen Verbindung der allgemeinen Formel (I) mit dem mindestens einen Carbonsäureanhydrid der allgemeinen Formel (II) bei Temperaturen im Bereich von 60 bis 150 °C, insbesondere im Bereich von 70 bis 120 °C, vorzugsweise im Bereich von 80 bis 100 °C, durchgeführt werden. Dennoch kann es einzelfallbedingt oder anwendungsbezogen erforderlich sein, von den vorgenannten Werten abzuweichen, ohne dass der Rahmen der vorliegenden Erfindung verlassen ist.

Auch die Umsetzungsdrücke können in weiten Bereichen variieren: Insbesondere kann die Umsetzung der mindestens einen Verbindung der allgemeinen Formel (I) mit dem mindestens einen Carbonsäureanhydrid der allgemeinen Formel (II) bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden. Dennoch kann es einzelfallbedingt oder anwendungsbezogen erforderlich sein, von den vorgenannten Werten abzuweichen, ohne dass der Rahmen der vorliegenden Erfindung verlassen ist.

Bei dem erfindungsgemäßen Verfahren wird die Umsetzung der mindestens einen Verbindung der allgemeinen Formel (I) mit dem mindestens einen Carbonsäureanhydrid der allgemeinen Formel (II) in Abwesenheit von Lösemitteln und/oder ohne jedwedes Lösemittel durchgeführt. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Im Rahmen des erfindungsgemäßen Herstellungsverfahrens wird bei der Umsetzung der mindestens einen Verbindung der allgemeinen Formel (I) mit dem mindestens einen Carbonsäureanhydrid der allgemeinen Formel (II) gleichzeitig eine Verbindung gemäß der allgemeinen Formel (IV)

R⁵-C(O)-OH (IV)

wobei der Rest R⁵ die zuvor angegebene Bedeutung hat, gebildet. Daher kann es erfindungsgemäß insbesondere vorgesehen sein, dass die Verbindung gemäß der allgemeinen Formel (IV) während oder nach erfolgter Umsetzung, insbesondere nach erfolgter Umsetzung, entfernt wird, vorzugsweise destillativ.

Erforderlichenfalls kann sich der Umsetzung der mindestens einen Verbindung der allgemeinen Formel (I) mit dem mindestens einen Carbonsäureanhydrid der allgemeinen Formel (II) eine Aufreinigung anschließen, insbesondere mittels Destillation und/oder Chromatographie, vorzugsweise mittels Destillation.

Insbesondere können gegebenenfalls noch vorhandene Edukte und Reaktionsnebenprodukte, insbesondere Verbindungen gemäß der allgemeinen Formel (IV), abdestilliert werden.

Was das als Edukt eingesetzte Carbonsäureanhydrid der allgemeinen Formel (II) anbelangt, so können hier grundsätzlich kommerzielle bzw. handelsübliche Produkte zum Einsatz kommen (z. B. Essigsäureanhydrid) oder herkömmliche Synthesen für die Herstellung des Carbonsäureanhydrids der allgemeinen Formel (II) herangezogen werden.

Gemäß einer besonderen Ausführungsform der vorliegenden Erfindung ist es jedoch in bestimmten Fällen erfindungsgemäß bevorzugt, für die Herstellung des Carbonsäureanhydrids der allgemeinen Formel (II) wie folgt vorzugehen:
Für den Fall, dass in der allgemeinen Formel (II) die Reste R² und R³ voneinander verschieden sind, und/oder für den Fall, dass in der allgemeinen Formel (II) die Reste R² und R³ in der allgemeinen Formel (II) jeweils einen Alkylrest mit mehr als zwei Kohlenstoffatomen darstellen, ist bzw. wird das Carbonsäureanhydrid der allgemeinen Formel (II) erhältlich bzw. erhalten durch Umsetzung von Essigsäureanhydrid (Acetanhydrid) mit mindestens einer Carbonsäure der allgemeinen Formel (IV)

R⁵ - C(O) - OH (IV)

wobei der Rest R⁵ die zuvor angegebene Bedeutung hat, jedoch mit der Maßgabe, dass die Reste R² und R³ voneinander verschieden sind und/oder dass die Reste R² und R³, jeweils unabhängig voneinander, einen Alkylrest mit mehr als zwei Kohlenstoffatomen darstellen.

Bei dieser Ausführungsform kann die Umsetzung von Essigsäureanhydrid (Acetanhydrid) mit der mindestens einen Carbonsäure der allgemeinen Formel (IV) insbesondere entsprechend der Reaktionsgleichung 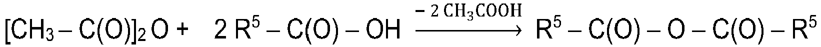 erfolgen.

Gemäß einer ersten Ausführungsform des zuvor beschriebenen Carbonsäureanhydrid-Herstellungsverfahrens kann ein symmetrisches Carbonsäureanhydrid der allgemeinen Formel (II) hergestellt werden. Insbesondere können gemäß dieser Ausführungsform in der allgemeinen Formel (II) die Reste R² und R³ identisch sein und vorzugsweise einen Alkylrest mit mehr als zwei Kohlenstoffatomen darstellen.

Gemäß einer zweiten, alternativen Ausführungsform des zuvor beschriebenen Carbonsäureanhydrid-Herstellungsverfahrens kann ein unsymmetrisches Carbonsäureanhydrid der allgemeinen Formel (II) hergestellt werden. Insbesondere können gemäß dieser alternativen Ausführungsform in der allgemeinen Formel (II) die Reste R² und R³ voneinander verschieden sein (vorzugsweise wobei in der allgemeinen Formel (II) die Reste R² und R³ jeweils einen Alkylrest mit mehr als zwei Kohlenstoffatomen darstellen).

Im Rahmen des zuvor beschriebenen Carbonsäureanhydrid-Herstellungsverfahrens kann die Umsetzung von Essigsäureanhydrid (Acetanhydrid) mit der mindestens einen Carbonsäure der allgemeinen Formel (IV) bei Temperaturen im Bereich von 60 bis 150 °C, insbesondere im Bereich von 70 bis 120 °C, vorzugsweise im Bereich von 80 bis 100 °C, durchgeführt werden. Dennoch kann es einzelfallbedingt oder anwendungsbezogen erforderlich sein, von den vorgenannten Werten abzuweichen, ohne dass der Rahmen der vorliegenden Erfindung verlassen ist.

Weiterhin kann im Rahmen des zuvor beschriebenen Carbonsäureanhydrid-Herstellungsverfahrens die Umsetzung von Essigsäureanhydrid (Acetanhydrid) mit der mindestens einen Carbonsäure der allgemeinen Formel (IV) bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden. Dennoch kann es einzelfallbedingt oder anwendungsbezogen erforderlich sein, von den vorgenannten Werten abzuweichen, ohne dass der Rahmen der vorliegenden Erfindung verlassen ist.

Im Rahmen des erfindungsgemäßen Herstellungsverfahrens werden als Reaktionsprodukt ein oder mehrere verkappte (blockierte) 3-Hydroxybuttersäuren und/oder deren Salze und/oder Ester der allgemeinen Formel (III)

CH₃-CH[O-C(O)R⁵]-CH₂-C(O)OR⁴ (III)

gebildet,
wobei in der allgemeinen Formel (III)
- der Rest R⁴ Methyl oder Ethyl, besonders bevorzugt Ethyl, oder ein Metallion, insbesondere Alkali- oder Erdalkalimetallion, darstellt,
- der Rest R⁵ ein lineares (geradkettiges) oder verzweigtes, gesättigtes oder ein- oder mehrfach ungesättigtes C₃-C₂₁-Alkyl, darstellt.

Insbesondere werden im Rahmen des erfindungsgemäßen Herstellungsverfahrens als Reaktionsprodukt ein oder mehrere verkappte (blockierte) 3-Hydroxybuttersäure-Salze und/oder -Ester der allgemeinen Formel (III)

CH₃-CH[O-C(O)R⁵]-CH₂-C(O)OR⁴ (III)

gebildet,
wobei in der allgemeinen Formel (III)
- der Rest R⁴ Methyl oder Ethyl, besonders bevorzugt Ethyl, oder ein Metallion, insbesondere Alkali- oder Erdalkalimetallion, darstellt,
- der Rest R⁵ ein lineares (geradkettiges) oder verzweigtes, gesättigtes oder ein- oder mehrfach ungesättigtes C₃-C₂₁-Alkyl, darstellt.

Gemäß einer besonderen Ausführungsform werden im Rahmen des erfindungsgemäßen Herstellungsverfahrens als Reaktionsprodukt ein oder mehrere verkappte (blockierte) 3-Hydroxybuttersäure-Ester der allgemeinen Formel (III)

CH₃-CH[O-C(O)R⁵]-CH₂-C(O)OR⁴ (III)

gebildet,
wobei in der allgemeinen Formel (III)
- der Rest R⁴ Methyl oder Ethyl, besonders bevorzugt Ethyl, darstellt,
- der Rest R⁵ ein lineares (geradkettiges) oder verzweigtes, gesättigtes oder ein- oder mehrfach ungesättigtes C₃-C₂₁-Alkyl, darstellt.

Gemäß einer weiteren besonderen Ausführungsform werden im Rahmen des erfindungsgemäßen Herstellungsverfahrens als Reaktionsprodukt ein oder mehrere verkappte (blockierte) 3-Hydroxybuttersäure-Ester der allgemeinen Formel (III)

CH₃-CH[O-C(O)R⁵]-CH₂-C(O)OR⁴ (III)

gebildet,
wobei in der allgemeinen Formel (III)
- der Rest R⁴ Ethyl darstellt,
- der Rest R⁵ ein lineares (geradkettiges) oder verzweigtes, gesättigtes oder ein- oder mehrfach ungesättigtes C₃-C₂₁-Alkyl, darstellt.

Wie zuvor bereits ausgeführt, wird das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*). Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Nach dem erfindungsgemäßen Verfahren sind als Reaktionsprodukt ein oder mehrere verkappte bzw. blockierte 3-Hydroxybuttersäuren und/oder deren Salze und/oder Ester oder deren Gemische erhältlich.

Insbesondere kann das Reaktionsprodukt ein oder mehrere verkappte (blockierte) 3-Hydroxybuttersäuren und/oder deren Salze und/oder Ester der allgemeinen Formel (III)

CH₃-CH[O-C(O)R⁵]-CH₂-C(O)OR⁴ (III)

umfassen,
wobei in der allgemeinen Formel (III)
- der Rest R⁴ Methyl oder Ethyl, besonders bevorzugt Ethyl, oder ein Metallion, insbesondere Alkali- oder Erdalkalimetallion, darstellt,
- der Rest R⁵ ein lineares (geradkettiges) oder verzweigtes, gesättigtes oder ein- oder mehrfach ungesättigtes C₃-C₂₁-Alkyl, darstellt.

Gemäß einer besonderen Ausführungsform kann das Reaktionsprodukt ein oder mehrere verkappte (blockierte) 3-Hydroxybuttersäure-Salze und/oder -Ester der allgemeinen Formel (III)

CH₃-CH[O-C(O)R⁵]-CH₂-C(O)OR⁴ (III)

umfassen,
wobei in der allgemeinen Formel (III)
- der Rest R⁴ Methyl oder Ethyl, besonders bevorzugt Ethyl, oder ein Metallion, insbesondere Alkali- oder Erdalkalimetallion, darstellt,
- der Rest R⁵ ein lineares (geradkettiges) oder verzweigtes, gesättigtes oder ein- oder mehrfach ungesättigtes C₃-C₂₁-Alkyl, darstellt.

Gemäß einer weiteren besonderen Ausführungsform kann das Reaktionsprodukt ein oder mehrere verkappte (blockierte) 3-Hydroxybuttersäure-Ester der allgemeinen Formel (III)

CH₃-CH[O-C(O)R⁵]-CH₂-C(O)OR⁴ (III)

umfassen,
wobei in der allgemeinen Formel (III)
- der Rest R⁴ Methyl oder Ethyl, besonders bevorzugt Ethyl, darstellt,
- der Rest R⁵ ein lineares (geradkettiges) oder verzweigtes, gesättigtes oder ein- oder mehrfach ungesättigtes C₃-C₂₁-Alkyl, darstellt.

Gemäß einer wiederum weiteren besonderen Ausführungsform kann das Reaktionsprodukt ein oder mehrere verkappte (blockierte) 3-Hydroxybuttersäure-Ester der allgemeinen Formel (III)

CH₃-CH[O-C(O)R⁵]-CH₂-C(O)OR⁴ (III)

umfassen,
wobei in der allgemeinen Formel (III)
- der Rest R⁴ Ethyl darstellt,
- der Rest R⁵ ein lineares (geradkettiges) oder verzweigtes, gesättigtes oder ein- oder mehrfach ungesättigtes C₃-C₂₁-Alkyl, darstellt.

Darüber hinaus kann das Reaktionsprodukt der allgemeinen Formel (III) mindestens zwei voneinander verschiedene Verbindungen der allgemeinen Formel (III) umfassen.

Insbesondere kann das Reaktionsprodukt der allgemeinen Formel (III) mindestens drei voneinander verschiedene Verbindungen der allgemeinen Formel (III) umfassen.

Somit ist nach dem erfindungsgemäßen Verfahren eine verkappte (blockierte) 3-Hydroxybuttersäure und/oder deren Salz und/oder Ester erhältlich,
wobei die verkappte (blockierte) 3-Hydroxybuttersäure und/oder deren Salz und/oder Ester der allgemeinen Formel (III)

   CH₃-CH[O-C(O)R⁵]-CH₂-C(O)OR⁴ (III)

   entspricht,
wobei in der allgemeinen Formel (III)
   - der Rest R⁴ Methyl oder Ethyl, besonders bevorzugt Ethyl, oder ein Metallion, insbesondere Alkali- oder Erdalkalimetallion, darstellt,
   - der Rest R⁵ ein lineares (geradkettiges) oder verzweigtes, gesättigtes oder ein- oder mehrfach ungesättigtes C₃-C₂₁-Alkyl, darstellt.

Insbesondere ist nach dem erfindungsgemäßen Verfahren ein Salz und/oder Ester einer verkappten (blockierten) 3-Hydroxybuttersäure, insbesondere wie zuvor definiert, erhältlich,
wobei das Salz und/oder der Ester der verkappten (blockierten) 3-Hydroxybuttersäure der allgemeinen Formel (III)

   CH₃-CH[O-C(O)R⁵]-CH₂-C(O)OR⁴ (III)

   entspricht,
wobei in der allgemeinen Formel (III)
   - der Rest R⁴ Methyl oder Ethyl, besonders bevorzugt Ethyl, oder ein Metallion, insbesondere Alkali- oder Erdalkalimetallion, darstellt,
   - der Rest R⁵ ein lineares (geradkettiges) oder verzweigtes, gesättigtes oder ein- oder mehrfach ungesättigtes C₃-C₂₁-Alkyl, darstellt.

Gemäß einer besonderen Ausführungsform ist nach dem erfindungsgemäßen Verfahren ein Ester einer verkappten (blockierten) 3-Hydroxybuttersäure, insbesondere wie zuvor definiert, erhältlich,
wobei der Ester der verkappten (blockierten) 3-Hydroxybuttersäure der allgemeinen Formel (III)

   CH₃-CH[O-C(O)R⁵]-CH₂-C(O)OR⁴ (III)

   entspricht,
wobei in der allgemeinen Formel (III)
   - der Rest R⁴ Methyl oder Ethyl, besonders bevorzugt Ethyl, darstellt,
   - der Rest R⁵ ein lineares (geradkettiges) oder verzweigtes, gesättigtes oder ein- oder mehrfach ungesättigtes C₃-C₂₁-Alkyl, darstellt.

Schließlich ist nach dem erfindungsgemäßen Verfahren ein Ester einer verkappten (blockierten) 3-Hydroxybuttersäure, insbesondere wie zuvor definiert, erhältlich,
wobei der Ester der verkappten (blockierten) 3-Hydroxybuttersäure der allgemeinen Formel (III)

   CH₃-CH[O-C(O)R⁵]-CH₂-C(O)OR⁴ (III)

   entspricht,
wobei in der allgemeinen Formel (III)
   - der Rest R⁴ Ethyl darstellt,
   - der Rest R⁵ ein lineares (geradkettiges) oder verzweigtes, gesättigtes oder ein- oder mehrfach ungesättigtes C₃-C₂₁-Alkyl, darstellt.

Weiterhin ist nach dem erfindungsgemäßen Verfahren ein Gemisch, welches mindestens zwei voneinander verschiedene verkappte (blockierte) 3-Hydroxybuttersäuren und/oder deren Salze und/oder Ester, wie zuvor definiert, umfasst, erhältlich.

Weiterhin ist nach dem erfindungsgemäßen Verfahren ein Gemisch, welches mindestens drei voneinander verschiedene verkappte (blockierte) 3-Hydroxybuttersäuren und/oder deren Salze und/oder Ester, wie zuvor definiert, umfasst, erhältlich.

Das nach dem erfindungsgemäßen Verfahren erhältliche Reaktionsprodukt, wie zuvor definiert, und/oder die nach dem erfindungsgemäßen Herstellungsverfahren erhältlichen verkappten bzw. blockierten 3-Hydroxybuttersäuren und/oder deren Salze und/oder Ester, wie zuvor definiert, und/oder das nach dem erfindungsgemäßen Herstellungsverfahren erhältliche Gemisch, wie zuvor definiert, weist gegenüber dem Stand der Technik eine Vielzahl von Vorteilen und Besonderheiten auf:
Wie die Anmelderin überraschend herausgefunden hat, eignet sich das nach dem erfindungsgemäßen Verfahren erhältliche Reaktionsprodukt, wie zuvor definiert, und/oder die nach dem erfindungsgemäßen Herstellungsverfahren erhältlichen verkappten bzw. blockierten 3-Hydroxybuttersäuren und/oder deren Salze und/oder Ester, wie zuvor definiert, und/oder das nach dem erfindungsgemäßen Herstellungsverfahren erhältliche Gemisch, wie zuvor definiert, insbesondere als Präkursor bzw. Metabolit von 3-Hydroxybuttersäure bzw. deren Salzen, da dieses bzw. diese einerseits physiologisch, insbesondere im Magen/Darm-Trakt, zu 3-Hydroxybuttersäure bzw. deren Salzen umgesetzt wird/werden und andererseits gleichzeitig eine gute physiologische Kompatibilität bzw. Verträglichkeit aufweist/aufweisen, insbesondere im Hinblick auf Nichttoxizität und akzeptable organoleptische Eigenschaften.

Darüber hinaus ist/sind das nach dem erfindungsgemäßen Verfahren erhältliche Reaktionsprodukt, wie zuvor definiert, und/oder die nach dem erfindungsgemäßen Herstellungsverfahren erhältlichen verkappten bzw. blockierten 3-Hydroxybuttersäuren und/oder deren Salze und/oder Ester, wie zuvor definiert, und/oder das nach dem erfindungsgemäßen Herstellungsverfahren erhältliche Gemisch, wie zuvor definiert, ohne Weiteres auf synthetischem Wege auch in großtechnischem Maßstab zugänglich bzw. verfügbar, und zwar auch mit der erforderlichen pharmazeutischen bzw. pharmakologischen Qualität.

Zudem kann/können das nach dem erfindungsgemäßen Verfahren erhältliche Reaktionsprodukt, wie zuvor definiert, und/oder die nach dem erfindungsgemäßen Herstellungsverfahren erhältlichen verkappten bzw. blockierten 3-Hydroxybuttersäuren und/oder deren Salze und/oder Ester, wie zuvor definiert, und/oder das nach dem erfindungsgemäßen Herstellungsverfahren erhältliche Gemisch, wie zuvor definiert, erforderlichenfalls in enantiomerenreiner bzw. enantiomerenangereicherter Form bereitgestellt werden.

Das nach dem erfindungsgemäßen Verfahren erhältliche Reaktionsprodukt, wie zuvor definiert, und/oder die nach dem erfindungsgemäßen Herstellungsverfahren erhältlichen verkappten bzw. blockierten 3-Hydroxybuttersäuren und/oder deren Salze und/oder Ester, wie zuvor definiert, und/oder das nach dem erfindungsgemäßen Herstellungsverfahren erhältliche Gemisch, wie zuvor definiert, stellt/stellen somit ein effizientes pharmakologisches Wirkstoff-Target im Rahmen einer Ketokörper-Therapie des menschlichen oder tierischen Körpers dar.

Nachfolgend werden noch die übrigen Erfindungsaspekte weiterführende erläutert und im Detail beschrieben.

Nachfolgend beschrieben ist weiterhin ein Verfahren zur Herstellung spezieller Carbonsäureanhydride, welche sich als Edukte für das erfindungsgemäße Herstellungsverfahren gemäß dem ersten Erfindungsaspekt eignen.

Zu diesem Zweck eignet sich ein Verfahren zur Herstellung eines Carbonsäureanhydrids der allgemeinen Formel (II)

R²-C(O)-O-C(O)-R³ (II)

wobei in der in der allgemeinen Formel (II) die Reste R²und R³, jeweils unabhängig voneinander, ein lineares (geradkettiges) oder verzweigtes, gesättigtes oder ein- oder mehrfach ungesättigtes C₃-C₂₁-Alkyl, darstellen, jedoch mit der Maßgabe, dass in der allgemeinen Formel (II) die Reste R²und R³voneinander verschieden sind und/oder dass in der allgemeinen Formel (II) die Reste R² und R³ jeweils einen Alkylrest mit mehr als zwei Kohlenstoffatomen darstellen,
durch Umsetzung von Essigsäureanhydrid (Acetanhydrid) mit mindestens einer Carbonsäure der allgemeinen Formel (IV)

R⁵-C(O)-OH (IV)

wobei der Rest R⁵ einen Rest R² oder R³ jeweils mit der zuvor angegebenen Bedeutung darstellt, jedoch mit der Maßgabe, dass die Reste R² und R³ voneinander verschieden sind und/oder dass die Reste R² und R³ jeweils einen Alkylrest mit mehr als zwei Kohlenstoffatomen darstellen.

Im Rahmen des Carbonsäureanhydrid-Herstellungsverfahrens kann die Umsetzung von Essigsäureanhydrid (Acetanhydrid) mit der mindestens einen Carbonsäure der allgemeinen Formel (IV) insbesondere entsprechend der Reaktionsgleichung 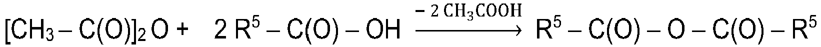 erfolgen (wobei der in der Reaktionsgleichung verwendete Rest die zuvor angegebene Bedeutung besitzt).

Gemäß einer besonderen Ausführungsform des Carbonsäureanhydrid-Herstellungsverfahrens wird ein symmetrisches Carbonsäureanhydrid der allgemeinen Formel (II) hergestellt. Bei dieser Ausführungsform sind in der vorstehenden allgemeinen Formel (II) die Reste R² und R³ identisch und stellen jeweils einen Alkylrest mit mehr als zwei Kohlenstoffatomen dar.

Gemäß einer weiteren besonderen, aber alternativen Ausführungsform des Carbonsäureanhydrid-Herstellungsverfahrens wird ein unsymmetrisches Carbonsäureanhydrid der allgemeinen Formel (II) hergestellt. Bei dieser Ausführungsform sind in der vorstehenden allgemeinen Formel (II) die Reste R² und R³ voneinander verschieden sind; vorzugsweise stellen in der allgemeinen Formel (II) die Reste R² und R³ jeweils einen Alkylrest mit mehr als zwei Kohlenstoffatomen dar.

Das Carbonsäureanhydrid-Herstellungsverfahren wird anhand des nachfolgenden Reaktionsschemas beispielhaft für die Umsetzung einer C₃-C₃₁-Alkylmonocarbonsäure mit Essigsäureanhydrid dargestellt (wobei die gleichermaßen gebildete Essigsäure der Einfachheit halber nicht dargestellt ist):

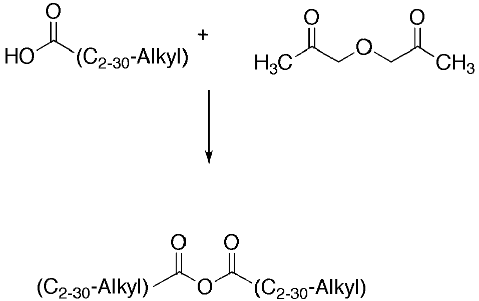

Im Rahmen des zuvor beschriebenen Carbonsäureanhydrid-Herstellungsverfahrens kann die Umsetzung von Essigsäureanhydrid (Acetanhydrid) mit der mindestens einen Carbonsäure der allgemeinen Formel (IV) bei Temperaturen im Bereich von 60 bis 150 °C, insbesondere im Bereich von 70 bis 120 °C, vorzugsweise im Bereich von 80 bis 100 °C, durchgeführt werden. Dennoch kann es einzelfallbedingt oder anwendungsbezogen erforderlich sein, von den vorgenannten Werten abzuweichen, ohne dass der Rahmen der vorliegenden Erfindung verlassen ist.

Weiterhin kann im Rahmen des zuvor beschriebenen Carbonsäureanhydrid-Herstellungsverfahrens die Umsetzung von Essigsäureanhydrid (Acetanhydrid) mit der mindestens einen Carbonsäure der allgemeinen Formel (IV) bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden. Dennoch kann es einzelfallbedingt oder anwendungsbezogen erforderlich sein, von den vorgenannten Werten abzuweichen, ohne dass der Rahmen der vorliegenden Erfindung verlassen ist.

Insbesondere wird im Rahmen des zuvor beschriebenen Carbonsäureanhydrid-Herstellungsverfahrens die Umsetzung (d. h. Umsetzung von Essigsäureanhydrid (Acetanhydrid) mit der mindestens einen Carbonsäure der allgemeinen Formel (IV)) in Abwesenheit von Lösemitteln und/oder ohne jedwedes Lösemittel durchgeführt. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Carbonsäureanhydrid-Herstellungsverfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Das Carbonsäureanhydrid-Herstellungsverfahren verläuft äußerst effizient, vor allem einstufig, und vor allem ohne die Bildung von störenden oder toxischen Nebenprodukten.

Im Unterschied hierzu verlaufen herkömmliche Carbonsäureanhydrid-Synthesen des Standes der Technik mehrstufig und typischerweise über die jeweiligen Carbonsäurechloride, welche zuvor in einem zusätzlichen Verfahrensschritt mittels Sulfurylchlorid aus den betreffenden Carbonsäuren hergestellt werden müssen, wobei die hierbei verwendeten Chemikalien bzw. Neben- und Zwischenprodukte zum Teil toxisch sind; zudem handelt es sich um eine mehrstufige Synthese, welche im großtechnischen Maßstab, insbesondere aus sicherheitstechnischen Gründen, nicht ohne Weiteres umzusetzen ist.

Hier bietet das Carbonsäureanhydrid-Herstellungsverfahren ein auch großtechnisch umzusetzende, effiziente wie einfache Alternative.

Das Carbonsäureanhydrid-Herstellungsverfahren stellt daher erstmals diese neue wie effiziente Syntheseroute für die speziellen zuvor definierten Carbonsäureanhydride bereit.

Das nach dem erfindungsgemäßen Herstellungsverfahren erhältliche Reaktionsprodukt, wie zuvor definiert, und/oder die nach dem erfindungsgemäßen Herstellungsverfahren erhältliche verkappte (blockierte) 3-Hydroxybuttersäure oder deren Salz oder Ester, wie zuvor definiert, und/oder das nach dem erfindungsgemäßen Herstellungsverfahren erhältliche Gemisch, wie zuvor definiert, eignet sich für eine pharmazeutische Zusammensetzung, insbesondere ein Arzneimittel oder Medikament, welche(s) ein nach dem erfindungsgemäßen Herstellungsverfahren erhältliches Reaktionsprodukt, wie zuvor definiert, und/oder eine nach dem erfindungsgemäßen Herstellungsverfahren erhältliche verkappte (blockierte) 3-Hydroxybuttersäure oder deren Salz oder Ester, wie zuvor definiert, und/oder ein nach dem erfindungsgemäßen Herstellungsverfahren erhältliches Gemisch, wie zuvor definiert, umfasst.

Insbesondere eignet sich das nach dem erfindungsgemäßen Herstellungsverfahren erhältliche Reaktionsprodukt, wie zuvor definiert, und/oder die nach dem erfindungsgemäßen Herstellungsverfahren erhältliche verkappte (blockierte) 3-Hydroxybuttersäure oder deren Salz oder Ester, wie zuvor definiert, und/oder das nach dem erfindungsgemäßen Herstellungsverfahren erhältliche Gemisch, wie zuvor definiert, für eine pharmazeutische Zusammensetzung zur prophylaktischen und/oder therapeutischen Behandlung bzw. zur Verwendung bei der prophylaktischen und/oder therapeutischen Behandlung von Erkrankungen des menschlichen oder tierischen Körpers. Hierbei kann es sich insbesondere um Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, handeln.

Das nach dem erfindungsgemäßen Herstellungsverfahren erhältliche Reaktionsprodukt, wie zuvor definiert, und/oder die nach dem erfindungsgemäßen Herstellungsverfahren erhältliche verkappte (blockierte) 3-Hydroxybuttersäure oder deren Salz oder Ester, wie zuvor definiert, und/oder das nach dem erfindungsgemäßen Herstellungsverfahren erhältliche Gemisch, wie zuvor definiert, eignet sich weiterhin zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen eignet sich das nach dem erfindungsgemäßen Herstellungsverfahren erhältliche Reaktionsprodukt, wie zuvor definiert, und/oder die nach dem erfindungsgemäßen Herstellungsverfahren erhältliche verkappte (blockierte) 3-Hydroxybuttersäure oder deren Salz oder Ester, wie zuvor definiert, und/oder das nach dem erfindungsgemäßen Herstellungsverfahren erhältliche Gemisch, wie zuvor definiert, für die Verwendung zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen eignet sich das nach dem erfindungsgemäßen Herstellungsverfahren erhältliche Reaktionsprodukt, wie zuvor definiert, und/oder die nach dem erfindungsgemäßen Herstellungsverfahren erhältliche verkappte (blockierte) 3-Hydroxybuttersäure oder deren Salz oder Ester, wie zuvor definiert, und/oder das nach dem erfindungsgemäßen Herstellungsverfahren erhältliche Gemisch, wie zuvor definiert, für die Verwendung zur prophylaktischen und/oder therapeutischen Behandlung bzw. zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung.

Gleichermaßen eignet sich das nach dem erfindungsgemäßen Herstellungsverfahren erhältliche Reaktionsprodukt, wie zuvor definiert, und/oder die nach dem erfindungsgemäßen Herstellungsverfahren erhältliche verkappte (blockierte) 3-Hydroxybuttersäure oder deren Salz oder Ester, wie zuvor definiert, und/oder das nach dem erfindungsgemäßen Herstellungsverfahren erhältliche Gemisch, wie zuvor definiert, für ein Nahrungsmittel- und/oder Lebensmittelerzeugnis, welches ein nach dem erfindungsgemäßen Herstellungsverfahren erhältliches Reaktionsprodukt, wie zuvor definiert, und/oder eine nach dem erfindungsgemäßen Herstellungsverfahren erhältliche verkappte (blockierte) 3-Hydroxybuttersäure oder deren Salz oder Ester, wie zuvor definiert, und/oder ein nach dem erfindungsgemäßen Herstellungsverfahren erhältliches Gemisch, wie zuvor definiert, umfasst.

Gemäß einer besonderen Ausführungsform kann das Nahrungsmittel- und/oder Lebensmittelerzeugnis insbesondere ein Nahrungsergänzungsmittel, ein funktionelles Lebensmittel (*Functional Food*)*,* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Schließlich eignet sich das nach dem erfindungsgemäßen Herstellungsverfahren erhältliche Reaktionsprodukt, wie zuvor definiert, und/oder die nach dem erfindungsgemäßen Herstellungsverfahren erhältliche verkappte (blockierte) 3-Hydroxybuttersäure oder deren Salz oder Ester, wie zuvor definiert, und/oder das nach dem erfindungsgemäßen Herstellungsverfahren erhältliche Gemisch, wie zuvor definiert, für die Verwendung in einem Nahrungsmittel- und/oder Lebensmittelerzeugnis.

Gemäß diesem Erfindungsaspekt kann das Nahrungsmittel- und/oder Lebensmittelerzeugnis insbesondere ein Nahrungsergänzungsmittel, ein funktionelles Lebensmittel (*Functional Food*)*,* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Weitere Ausgestaltungen, Abwandlungen und Variationen der vorliegenden Erfindung sind für den Fachmann beim Lesen der Beschreibung ohne Weiteres erkennbar oder realisierbar, ohne dass er dabei den Rahmen der vorliegenden Erfindung verlässt.

Die vorliegende Erfindung wird anhand der nachfolgenden Ausführungsbeispiele veranschaulicht, welche die vorliegende Erfindung jedoch keinesfalls beschränken sollen, sondern lediglich die beispielhafte und nichtlimitierende Durchführungsweise und Ausgestaltung der vorliegenden Erfindung erläutern sollen.

### AUSFÜHRUNGSBEISPIELE:

### Herstellungsbeispiele

Das erfindungsgemäße Herstellungsverfahren wird anhand der nachfolgenden Ausführungsbeispiele veranschaulicht. Die diesbezüglichen Reaktionsschemata sind im allgemeinen Beschreibungsteil dargestellt und erläutert.

### Herstellung von 3-Acetoxybuttersäureethylester (3-Ac-BHB-Ethylester) und Anwendungsversuche

In einem 250-ml-Mehrhalskolben mit Dephlegmator (Partialkondensator) und Destillationsbrücke werden 50 g (R)/(S)-3-Hydroxybuttersäureethylester (racemischer 3-BHB-Ethylester) und 55 g Essigsäureanhydrid vorgelegt. Das Reaktionsgemisch wird bei 100 °C unter Rühren und unter Rückfluss für 10 h zur Reaktion gebracht. Danach werden die entstandene Essigsäure sowie überschüssiges Essigsäureanhydrid unter Vakuum abdestilliert. Es wird ein 3-Acetoxybuttersäureethylester mit 98 % Reinheit erhalten.

Die Charakterisierung erfolgt mittels Gaschromatographie (GC) und GC-MS-Analyse (Gaschromatographie mit Massenspektrometrie-Kopplung).

Die Ergebnisse des Umsatz/Zeit-Verlaufs sind in der nachfolgenden Tabelle zusammengefasst:

| **Zeit / h** | **Essigsäureanhydrid / %** | **3-BHB-Ethylester / %** | **3-Ac-BHB-Ethylester (Produkt) / %** | **unbekannt / %** | **Kommentar** |
|---|---|---|---|---|---|
| 1 | 28,8 | 38,9 | 31,3 | 1 | 80 °C |
| 3 | 23,1 | 26,1 | 49,5 | 1,3 | - |
| 6 | 18,6 | 15,9 | 63,9 | 1,6 | Temperaturerhöhung auf 100 °C |
| 9 | 12,4 | 3,2 | 82,6 | 1,8 | - |
| 12 | 11,5 | 1,3 | 85,6 | 1,6 | - |
| - | 0 | 0,6 | 97,7 | 1,7 | nach Destillation |

Der Geschmack des 3-Acetoxybuttersäureethylesters ist deutlich weniger unangenehm und bitter als der des reinen 3-BHB-Ethylesters oder gar vom 3-BHB-Triglycerid.

Spaltungsversuche (Spaltversuche) mit 3-Acetoxybuttersäureethylester in einem Magen- oder Darmmedium (FaSSGF-Medium, welches den Magen simuliert, oder FaSSIF-Medium, welches den Darmtrakt simuliert), jeweils in Anwesenheit oder in Abwesenheit von Pankreatin, belegen die Spaltung zu 3-BHB in freier Form. Diese Spaltungsversuche belegen, dass verkappte (blockierte) 3-Hydroxybuttersäure bzw. deren Salze oder Ester, hier konkret 3-Acetoxybuttersäureethylester, effiziente Präkursoren bzw. Metabolite der freien Hydroxybuttersäure bzw. deren Salze darstellen, insbesondere im Hinblick auf ihre beabsichtigte Wirkung, welche zudem in physiologisch verträglicher bzw. physiologisch kompatibler Form vorliegen.

### Weitere Umsetzung von 3-Acetoxybuttersäureethylester (3-Ac-BHB-Ethylester) und Anwendungsversuche

Im Weiteren kann gezeigt werden, dass der auf diese Weise erhaltene 3-Acetoxybuttersäureethylester mit Enzym als Katalysator (z. B. immobilisiertes Enzym, wie z. B. CALB-Lipase auf Polymerträger, abgeleitet von *Candida antarctica,* z. B. Novozym^{®}435 von der Fa. Sigma-Aldrich bzw. Merck oder Lipozym^{®}435 von der Fa. Strem Chemicals, Inc.) in einer Umesterung zu Glyceriden als Edukt eigesetzt werden kann (Umsetzung bei 50 bis 70 °C, 24 h 1 Gew.-% Enzym). Als weiteres Edukt wird Triacetin eingesetzt; da dieses bereits Acetylgruppen enthält, bilden sich bei einer etwaigen Umesterung an der bereits acetylierten OH-Gruppe des 3-BHB-Ethylesters keine unerwünschten Nebenprodukte. Als Koppelprodukt bildet sich nur Essigsäureethylester (Ethylacetat), welcher ohne Weiteres entfernt werden kann. Es entsteht ein Gemisch von Mono-, Di- und Triglyceriden der 3-Acetoxybuttersäure.

Spaltungsversuche (Spaltversuche) dieses Gemischs in einem Magen- oder Darmmedium (FaSSGF-Medium, welches den Magen simuliert, oder FaSSIF-Medium, welches den Darmtrakt simuliert), jeweils in Anwesenheit oder in Abwesenheit von Pankreatin, belegen die Spaltung zu 3-BHB in freier Form (Spaltungskaskade von Triglycerid über Diglycerid zu Monoglycerid zu freier 3-BHB). Diese Spaltungsversuche belegen, dass auch die Glyceride der verkappten (blockierten) 3-Hydroxybuttersäure bzw. deren Salze effiziente Präkursoren bzw. Metabolite der freien 3-Hydroxybuttersäure bzw. deren Salze darstellen, insbesondere im Hinblick auf ihre beabsichtigte Wirkung, welche zudem in physiologisch verträglicher bzw. physiologisch kompatibler Form vorliegen.

Dies zeigt, dass verkappte (blockierte) 3-Hydroxybuttersäure bzw. deren Salze oder Ester nicht nur selbst effiziente Präkursoren bzw. Metabolite der freien 3-Hydroxybuttersäure bzw. deren Salze darstellen, sondern auch als Edukte für die Synthese weiterer Präkursoren bzw. Metabolite der freien 3-Hydroxybuttersäure bzw. deren Salze verwendet werden können.

### Weitere Herstellungsbeispiele und Anwendunasversuche

Nach dem zuvor beschriebenen Carbonsäureanhydrid-Herstellungsverfahren werden zunächst jeweils die Carbonsäureanhydride von Heptansäure (C₇-Säure), Laurinsäure (C₁₂-Säure) und Ölsäure (C₁₈-Säure) hergestellt.

Für die Herstellung des Carbonsäureanhydrids von Heptansäure (C₇-Säure) werden in einem 2.000-ml-Mehrhalskolben mit Dephlegmator (Partialkondensator) und Destillationsbrücke 860 g Heptansäure vorgelegt und bei 90 °C 445 g Essigsäureanhydrid unter Rühren zugetropft. Das Reaktionsgemisch wird danach bei 130 °C unter Rückfluss für 6 h unter Rühren zur Reaktion gebracht. Danach werden die entstandene Essigsäure sowie das überschüssige Essigsäureanhydrid unter Vakuum abdestilliert. Es wird das Heptansäureanhydrid erhalten. Die Charakterisierung erfolgt mittels GC und GC-MS.

In entsprechender Weise werden die Carbonsäureanhydride von Laurinsäure (C₁₂-Säure) und Ölsäure (C₁₈-Säure) hergestellt.

Nachfolgend wird - entsprechend der zuvor beschriebenen Herstellung von 3-Acetoxybuttersäureethylester - das betreffende Carbonsäureanhydrid mit 3-BHB-Ethylester umgesetzt, so dass jeweils die in 3-Position mit dem Carbonsäureanhydrid verkappten 3-BHB-Ethylester resultieren.

Spaltungsversuche (Spaltversuche) mit diesen verkappten 3-BHB-Ethylestern jeweils in einem Magen- oder Darmmedium (FaSSGF-Medium, welches den Magen simuliert, oder FaSSIF-Medium, welches den Darmtrakt simuliert), jeweils in Anwesenheit oder in Abwesenheit von Pankreatin, belegen die Spaltung zu 3-BHB in freier Form. Diese Spaltungsversuche belegen, dass verkappte (blockierte) 3-Hydroxybuttersäure bzw. deren Salze oder Ester effiziente Präkursoren bzw. Metabolite der freien 3-Hydroxybuttersäure bzw. deren Salze darstellen, insbesondere im Hinblick auf ihre beabsichtigte Wirkung, welche zudem in physiologisch verträglicher bzw. physiologisch kompatibler Form vorliegen.

### Weitere physiologische Anwendunasversuche: in-vitro-Verdauversuche

### Verdauversuche (Spalt- bzw. Sipaltungsversuche) von erfindunasaemäß erhältlichen Verbindungen

Mittels Spaltungsversuchen wird gezeigt, dass erfindungsgemäß hergestellte Reaktionsprodukte bzw. deren Gemische im menschlichen gastrointestinalen Trakt gespalten werden können. Als Ausgangsgemisch werden einerseits ein nach dem erfindungsgemäßen Verfahren wie zuvor beschrieben erhaltener und aufgereinigter 3-Acetoxybuttersäureethylester und andererseits jeweils die nach dem erfindungsgemäßen Verfahren wie zuvor beschrieben erhaltenen und aufgereinigten, in 3-Position mit dem Carbonsäureanhydrid (Heptansäureanhydrid, Laurinsäureanhydrid bzw. Ölsäureanhydrid) verkappten 3-BHB-Ethylester eingesetzt.

Für die Spaltungsversuche unter körpernahen Bedingungen werden zwei Medien untersucht:
- FaSSGF, welches den Magen simuliert
- FaSSIF, welches den Darmtrakt simuliert

Beide Medien stammen von der Firma Biorelevant^{®}, Ltd., Großbritannien. Zusätzlich wird in einigen Experimenten beiden Medien jeweils Schweine-Pankrease zugesetzt (Panzytrat^{®}40.000, Fa. Allergan).

Die Ergebnisse der Spaltungsversuche in einem FaSSGF- bzw. FaSSIF-Medium mit Panzytrat^{®} und ohne Panzytrat^{®} (jeweils 35 °C, 24 h) zeigen, dass alle Proben unter FaSSGF-Bedingungen mit Panzytrat^{®} und ohne Panzytrat^{®} hydrolysieren; dies liegt hauptsächlich am niedrigen pH-Wert (pH = 1,6). Bei FaSSIF-Bedingungen findet eine geringere Umsetzung unter Verwendung von Panzytrat^{®} statt.

Bei allen Experimenten ist zu erkennen, dass die gewünschte freien Säure 3-BHB generiert wird. Der Umsatz/Zeit-Verlauf der wässrigen Spaltung der erfindungsgemäße hergestellten Verbindungen, einschließlich Zunahme der Säurezahl über die Zeit, belegt die gewünschte Zersetzung der Edukte zu der freien Säure. Dies wird durch entsprechende Analytik bestätigt.

Die zuvor geschilderten Spaltungsversuche belegen, dass verkappte (blockierte) 3-Hydroxybuttersäure bzw. deren Salze oder Ester effiziente Präkursoren bzw. Metabolite der freien Hydroxybuttersäure bzw. deren Salze darstellen, insbesondere im Hinblick auf ihre beabsichtigte Wirkung, und zudem in physiologisch verträglicher bzw. physiologisch kompatibler Form vorliegen.

## Patentansprüche

1. Verfahren zur Herstellung von verkappter 3-Hydroxybuttersäure oder deren Salzen oder Estern,
wobei mindestens eine Verbindung der allgemeinen Formel (I)
CH₃-CH(OH)-CH₂-C(O)OR¹ (I)
wobei in der allgemeinen Formel (I) der Rest R¹ Wasserstoff oder Methyl oder Ethyl darstellt,
mit mindestens einem Carbonsäureanhydrid der allgemeinen Formel (II)
R²-C(O)-O-C(O)-R³ (II)
wobei in der in der allgemeinen Formel (II) die Reste R² und R³, jeweils unabhängig voneinander, ein lineares oder verzweigtes, gesättigtes oder ein- oder mehrfach ungesättigtes C₃-C₂₁-Alkyl darstellen,
umgesetzt wird, gegebenenfalls gefolgt von einer Hydrolyse für den Fall, dass R¹ Wasserstoff darstellt,
wobei die Umsetzung der mindestens einen Verbindung der allgemeinen Formel (I) mit dem mindestens einen Carbonsäureanhydrid der allgemeinen Formel (II) in Abwesenheit von Lösemitteln und/oder ohne jedwedes Lösemittel durchgeführt wird,
so dass als Reaktionsprodukt ein oder mehrere verkappte 3-Hydroxybuttersäuren und/oder deren Salze und/oder Ester der allgemeinen Formel (III)
CH₃-CH[O-C(O)R⁵]-CH₂-C(O)OR⁴ (III)
wobei in der allgemeinen Formel (III) der Rest R⁴ Methyl oder Ethyl oder ein Metallion, insbesondere Alkali- oder Erdalkalimetallion, darstellt und der Rest R⁵ einen Rest R² oder R³ jeweils mit der zuvor angegebenen Bedeutung darstellt,
erhalten wird/werden.

2. Verfahren nach Anspruch 1,
wobei Verbindung der allgemeinen Formel (I) in racemischer Form oder in Form des (R)-Enantiomers eingesetzt wird.

3. Verfahren nach Anspruch 1 oder Anspruch 2,
wobei in der allgemeinen Formel (I) der Rest R¹ Ethyl darstellt und/oder wobei als Verbindung der allgemeinen Formel (I) 3-Hydroxybuttersäureethylester (Ethyl-3-hydroxybutyrat) der Formel CH₃-CH(OH)-CH₂-C(O)OC₂H₅ eingesetzt wird.

4. Verfahren nach einem der vorangehenden Ansprüche,
wobei die Hydrolyse sauer oder basisch, insbesondere basisch und/oder in Gegenwart von Metallionen, insbesondere Alkali- oder Erdalkalimetallionen, durchgeführt wird.

5. Verfahren nach einem der vorangehenden Ansprüche,
wobei als Carbonsäureanhydrid der allgemeinen Formel (II) eine Verbindung eingesetzt wird, bei welcher die Reste R² und R³ identisch sind, und/oder wobei als Carbonsäureanhydrid der allgemeinen Formel (II) ein symmetrisches Carbonsäureanhydrid eingesetzt wird; oder aber
wobei als Carbonsäureanhydrid der allgemeinen Formel (II) eine Verbindung eingesetzt wird, bei welcher die Reste R² und R³ voneinander verschieden sind, und/oder wobei als Carbonsäureanhydrid der allgemeinen Formel (II) ein unsymmetrisches Carbonsäureanhydrid eingesetzt wird.

6. Verfahren nach einem der vorangehenden Ansprüche,
wobei die Umsetzung der mindestens einen Verbindung der allgemeinen Formel (I) mit dem mindestens einen Carbonsäureanhydrid der allgemeinen Formel (II) bei Temperaturen im Bereich von 60 bis 150 °C, insbesondere im Bereich von 70 bis 120 °C, vorzugsweise im Bereich von 80 bis 100 °C, durchgeführt wird; und/oder
wobei die Umsetzung der mindestens einen Verbindung der allgemeinen Formel (I) mit dem mindestens einen Carbonsäureanhydrid der allgemeinen Formel (II) bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird.

7. Verfahren nach einem der vorangehenden Ansprüche,
wobei bei der Umsetzung der mindestens einen Verbindung der allgemeinen Formel (I) mit dem mindestens einen Carbonsäureanhydrid der allgemeinen Formel (II) gleichzeitig eine Verbindung gemäß der allgemeinen Formel (IV)
R⁵-C(O)-OH (IV)
wobei der Rest R⁵ die zuvor angegebene Bedeutung hat, gebildet wird;
insbesondere wobei die Verbindung gemäß der allgemeinen Formel (IV) während oder nach erfolgter Umsetzung, insbesondere nach erfolgter Umsetzung, entfernt wird, vorzugsweise destillativ.

8. Verfahren nach einem der vorangehenden Ansprüche,
wobei sich der Umsetzung der mindestens einen Verbindung der allgemeinen Formel (I) mit dem mindestens einen Carbonsäureanhydrid der allgemeinen Formel (II) eine Aufreinigung anschließt, insbesondere mittels Destillation und/oder Chromatographie, vorzugsweise mittels Destillation;
insbesondere wobei gegebenenfalls noch vorhandene Edukte und Reaktionsnebenprodukte, insbesondere Verbindungen gemäß der allgemeinen Formel (IV), abdestilliert werden.

9. Verfahren nach einem der vorangehenden Ansprüche,
wobei als Reaktionsprodukt ein oder mehrere verkappte 3-Hydroxybuttersäure-Ester der allgemeinen Formel (III)
CH₃-CH[O-C(O)R⁵]-CH₂-C(O)OR⁴ (III)
gebildet werden,
wobei in der allgemeinen Formel (III)
• der Rest R⁴ Methyl oder Ethyl darstellt,
• der Rest R⁵ ein lineares oder verzweigtes, gesättigtes oder ein- oder mehrfach ungesättigtes C₃-C₂₁-Alkyl darstellt.

10. Verfahren nach einem der vorangehenden Ansprüche,
wobei als Reaktionsprodukt ein oder mehrere verkappte 3-Hydroxybuttersäure-Ester der allgemeinen Formel (III)
CH₃-CH[O-C(O)R⁵]-CH₂-C(O)OR⁴ (III)
gebildet werden,
wobei in der allgemeinen Formel (III)
• der Rest R⁴ Ethyl darstellt,
• der Rest R⁵ ein lineares oder verzweigtes, gesättigtes oder ein- oder mehrfach ungesättigtes C₃-C₂₁-Alkyl darstellt.

## Claims

1. A method for producing capped (blocked) 3-hydroxybutyric acid or its salts or esters,
wherein at least one compound of the general formula (I)
CH₃-CH(OH)-CH₂-C(O)OR¹ (I)
wherein in the general formula (I) the radical R¹ represents hydrogen or methyl or ethyl, is reacted with at least one carboxylic acid anhydride of the general formula (II)
R²-C(O)-O-C(O)-R³ (II)
wherein in the general formula (II) the radicals R² and R³, each independently of one another, represent a linear or branched, saturated or mono- or polyunsaturated C₃-C₂₁-alkyl,
optionally followed by hydrolysis in the case that R¹ represents hydrogen,
wherein the reaction of the at least one compound of the general formula (I) with the at least one carboxylic acid anhydride of the general formula (II) is carried out in the absence of solvents and/or without any solvent,
so that, as reaction product, there is/are obtained one or more capped (blocked) 3-hydroxybutyric acids and/or their salts and/or esters of the general formula (III)
CH₃-CH[O-C(O)R⁵]-CH₂-C(O)OR⁴ (III)
wherein in the general formula (III) the radical R⁴ represents methyl or ethyl or a metal ion, especially an alkali metal or alkaline earth metal ion, and the radical R⁵ represents a radical R² or R³ each having the meaning defined hereinabove.

2. The method according to claim 1,
wherein the compound of general formula (I) is used in racemic form or in the form of the (R)-enantiomer.

3. The method according to claim 1 or claim 2,
wherein in the general formula (I) the radical R¹ represents ethyl and/or
wherein, as a compound of the general formula (I), 3-hydroxybutyric acid ethyl ester (ethyl 3-hydroxybutyrate) of the formula CH₃-CH(OH)-CH₂-C(O)OC₂H₅ is used.

4. The method according to any of the preceding claims,
wherein the hydrolysis is carried out under acidic or basic conditions, especially under basic conditions, and/or in the presence of metal ions, especially alkali metal or alkaline earth metal ions.

5. The method according to any of the preceding claims,
wherein, as a carboxylic acid anhydride of the general formula (II), a compound in which the radicals R² and R³ are identical is used, and/or wherein, as a carboxylic acid anhydride of the general formula (II), a symmetrical carboxylic acid anhydride is used; or else
wherein, as a carboxylic acid anhydride of the general formula (II), a compound in which the radicals R² and R³ are different from one another is used, and/or wherein, as a carboxylic acid anhydride of the general formula (II), an asymmetric carboxylic acid anhydride is used.

6. The method according to any of the preceding claims,
wherein the reaction of the at least one compound of the general formula (I) with the at least one carboxylic acid anhydride of the general formula (II) is carried out at temperatures in the range of from 60 to 150 °C, especially in the range of from 70 to 120 °C, preferentially in the range of from 80 to 100 °C; and/or
wherein the reaction of the at least one compound of the general formula (I) with the at least one carboxylic acid anhydride of the general formula (II) is carried out at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at about 1 bar.

7. The method according to any of the preceding claims,
wherein during the reaction of the at least one compound of the general formula (I) with the at least one carboxylic acid anhydride of the general formula (II), a compound of the general formula (IV)
R⁵-C(O)-OH (IV)
is formed simultaneously, wherein the radical R⁵ has the meaning defined hereinabove;
especially wherein the compound of the general formula (IV) is withdrawn during or after the reaction has taken place, especially after the reaction has taken place, preferentially by distillation.

8. The method according to any of the preceding claims,
wherein the reaction of the at least one compound of the general formula (I) with the at least one carboxylic acid anhydride of the general formula (II) is followed by purification, especially by means of distillation and/or chromatography, preferentially by means of distillation;
especially wherein any starting compounds and reaction by-products still present, especially compounds of the general formula (IV), are distilled off.

9. The method according to any of the preceding claims,
wherein, as a reaction product, one or more capped (blocked) 3-hydroxybutyric acid salts and/or esters of the general formula (III)
CH₃-CH[O-C(O)R⁵]-CH₂-C(O)OR⁴ (III)
is/are formed,
wherein in the general formula (III)
• the radical R⁴ represents methyl or ethyl,
• the radical R⁵ represents a linear or branched, saturated or mono- or polyunsaturated C₃-C₂₁-alkyl.

10. The method according to any of the preceding claims,
wherein, as a reaction product, one or more capped (blocked) 3-hydroxybutyric acid salts and/or esters of the general formula (III)
CH₃-CH[O-C(O)R⁵]-CH₂-C(O)OR⁴ (III)
is/are formed,
wherein in the general formula (III)
• the radical R⁴ represents ethyl,
• the radical R⁵ represents a linear or branched, saturated or mono- or polyunsaturated C₃-C₂₁-alkyl.

## Revendications

1. Procédé de préparation de l'acide 3-hydroxybutyrique coiffé (bloqué) ou de ses sels ou esters,
où au moins un composé de formule générale (I)
CH₃-CH(OH)-CH₂-C(O)OR¹ (I)
où, dans la formule générale (I), le radical R¹ représente l'hydrogène ou le méthyle ou l'éthyle,
avec au moins un anhydride d'acide carboxylique de formule générale (II)
R²-C(O)-O-C(O)-R³ (II)
où, dans la formule générale (II), les radicaux R² et R³ représentent, chacun indépendamment l'un de l'autre, un groupe alkyle en C₃-C₂₁ , linéaire ou ramifié, saturé ou mono- ou polyinsaturé,
le cas échéant suivie d'une hydrolyse dans le cas où R¹ représente un atome d'hydrogène,
où la réaction dudit au moins un composé de formule générale (I) avec ledit au moins un anhydride d'acide carboxylique de formule générale (II) est réalisée en l'absence de solvants et/ou sans aucun solvant,
de sorte que l'on obtient, comme produit de réaction, un ou plusieurs acides 3-hydroxybutyriques coiffés (bloqués) et/ou leurs sels et/ou esters de formule générale (III)
CH₃-CH[O-C(O)R⁵]-CH₂-C(O)OR⁴ (III)
où, dans la formule générale (III), le radical R⁴ représente un groupe méthyle ou éthyle ou un ion métallique, en particulier un ion de métal alcalin ou alcalino-terreux, et le radical R⁵ représente un radical R² ou R³ ayant chacun la signification indiquée précédemment.

2. Procédé selon la revendication 1,
où le composé de formule générale (I) est utilisé sous forme racémique ou sous forme de l'énantiomère (R).

3. Procédé selon la revendication 1 ou la revendication 2,
où, dans la formule générale (I), le radical R représente¹ éthyle et/ou
où l'on utilise, comme composé de formule générale (I), l'ester éthylique de l'acide 3-hydroxybutyrique (3-hydroxybutyrate d'éthyle) de formule CH₃-CH(OH)-CH₂-C(O)OC₂H₂₅.

4. Procédé selon l'une quelconque des revendications précédentes,
où l'hydrolyse est réalisée de manière acide ou basique, en particulier de manière basique, et/ou en présence d'ions métalliques, en particulier d'ions de métaux alcalins ou alcalino-terreux.

5. Procédé selon l'une quelconque des revendications précédentes,
où l'on utilise, comme anhydride d'acide carboxylique de formule générale (II), un composé où les restes R² et R³ sont identiques et/ou où l'on utilise, comme anhydride d'acide carboxylique de formule générale (II), un anhydride d'acide carboxylique symétrique; ou bien
où l'on utilise, comme anhydride d'acide carboxylique de formule générale (II), un composé où les radicaux R² et R³ sont différents l'un de l'autre, et/ou où l'on utilise, comme anhydride d'acide carboxylique de formule générale (II), un anhydride d'acide carboxylique asymétrique.

6. Procédé selon l'une quelconque des revendications précédentes,
où la réaction dudit au moins un composé de formule générale (I) avec ledit au moins un anhydride d'acide carboxylique de formule générale (II) est effectuée à des températures dans la plage de 60 à 150 °C, en particulier dans la plage de 70 à 120 °C, de préférence dans la plage de 80 à 100 °C; et/ou
où la réaction dudit au moins un composé de formule générale (I) avec ledit au moins un anhydride d'acide carboxylique de formule générale (II) est réalisée à une pression dans la plage de 0,0001 bar à 10 bars, en particulier dans la plage de 0,001 bar à 5 bars, de préférence dans la plage de 0,01 bar à 2 bars, de manière particulièrement préférée dans la plage de 0,05 bar à 1 bar, tout particulièrement à environ 1 bar.

7. Procédé selon l'une quelconque des revendications précédentes,
où, lors de la réaction dudit au moins un composé de formule générale (I) avec ledit au moins un anhydride d'acide carboxylique de formule générale (II), on obtient simultanément un composé selon la formule générale (IV)
R⁵-C(O)-OH (IV)
où le radical R⁵ a la signification indiquée précédemment;
en particulier où le composé selon la formule générale (IV) est éliminé, de préférence par distillation, pendant ou après la réaction, en particulier après la réaction.

8. Procédé selon l'une quelconque des revendications précédentes,
où la réaction dudit au moins un composé de formule générale (I) avec ledit au moins un anhydride d'acide carboxylique de formule générale (II) est suivie d'une purification, en particulier par distillation et/ou chromatographie, de préférence par distillation;
en particulier en éliminant par distillation les matières de départ et les sous-produits de réaction le cas échéant encore présents, en particulier les composés selon la formule générale (IV).

9. Procédé selon l'une quelconque des revendications précédentes,
Où, comme produit de réaction, un ou plusieurs esters d'acide 3-hydroxybutyrique coiffés (bloqués) de formule générale (III)
CH₃-CH[O-C(O)R⁵]-CH₂-C(O)OR⁴ (III)
sont formés,
où dans la formule générale (III)
• le radical R⁴ représente un groupe méthyle ou éthyle,
• le radical R⁵ représente un groupe alkyle en C₃-C₂₁, linéaire ou ramifié, saturé ou mono- ou polyinsaturé.

10. Procédé selon l'une quelconque des revendications précédentes,
Où, comme produit de réaction, un ou plusieurs esters d'acide 3-hydroxybutyrique coiffés (bloqués) de formule générale (III)
CH₃-CH[O-C(O)R⁵]-CH₂-C(O)OR⁴ (III)
sont formés,
où dans la formule générale (III)
• le reste R⁴ représente un groupe éthyle,
• le radical R⁵ représente un groupe alkyle en C₃-C₂₁, linéaire ou ramifié, saturé ou mono- ou polyinsaturé.
